# EUROPEAN PATENT APPLICATION

(11) **EP 4 609 802 A1**
(43) Date of publication of application: **03.09.2025**
(21) Application number: 23893388.1
(22) Date of filing: 19.09.2023
(51) Int. Cl.: A61B 17/072

(54) **ANASTOMAT WITH SWITCH LOCKING LINKAGE MECHANISM**

(30) Priority: 21.11.2022 CN 202211452209
(71) Applicant: Innolcon Medical Technology (Suzhou) Co., Ltd., Jiangsu 215000 (CN); Innolcon Medical Technology (Hefei) Co., Ltd., Hefei, Anhui 230000 (CN)
(72) Inventor: HUANG, Aiyu, Suzhou, Jiangsu 215000 (CN); WU, Jun, Suzhou, Jiangsu 215000 (CN); LIU, Wei, Suzhou, Jiangsu 215000 (CN); LUO, Wei, Suzhou, Jiangsu 215000 (CN)
(74) Representative: Patentanwaltskanzlei WILHELM & BECK
(86) International application number: PCT/CN2023/119762
(87) International publication number: WO 2024/109295

(57) **Abstract**

Disclosed herein is a stapler with switch-lock linkage mechanism, the stapler includes a housing, a knob, an effector, a drive motor, a lead screw and a firing locking push button. The firing locking push button is inserted in the housing, with at least one end thereof protruded from the housing. A front end of the firing locking push button has a driving part, and a bottom of the driving part is fixedly arranged with a trigger block matching a detection module of the firing switch. A push rod is arranged in the housing in parallel with the lead screw, with a first end abutting against the driving part all the time. The firing locking push button drives, through radial movement thereof, the driving part to synchronously drive the push rod to move axially relative to the knob, such that a second end of the push rod is inserted into the knob to lock the knob; meanwhile, the trigger block leaves the detection module. In the present disclosure, a linkage structure is formed between the firing locking push button, the push rod and the detection module, triggering of the firing switch and locking of the knob can be completed simultaneously through radial movement of the firing locking push button, thereby ensuring the surgical accuracy and the use stability and convenience.

## Description

This disclosure claims priority to Chinese patent application No. 202211452209.2, titled "STAPLER WITH SWITCH-LOCK LINKAGE MECHANISM", filed on November 21, 2022 with the China National Intellectual Property Administration, the entire disclosure of which is incorporated herein by reference.

### FIELD

The present disclosure relates to the technical field of electric staplers, and in particular, to a stapler with switch-lock linkage mechanism.

### BACKGROUND

In minimally invasive surgery, the electric stapler is typically used for excision and closure of tissue. Laparoscopic stapler is a medical equipment which perform excision with a staple cartridge assembly clamping the lesion site and suture the lesion site at the same time.

As disclosed in patent application with publication number CN107714122A, rotation of end effector of existing electric laparoscopic cutting stapler or manual cutting stapler is mostly achieved by manually rotating the knob. In actual operation, a doctor generally has to manually rotate the staple cartridge assembly of the stapler to determine an appropriate position to be close to the tissue to be cut, and, during the process of cutting and closing the tissue, it is required that the position of the cutting assembly of the stapler be relatively fixed to reduce pulling of the head portion during the process of cutting and closing the tissue. The accuracy of manual control is limited, specifically, hand fatigue during long-term surgery may affect the accuracy of the knob control, which further affects the position accuracy of the cutting assembly of the end effector of the stapler, causing the position of the cutting assembly to shift, affecting the stapling effect of the anastomosis, resulting in problems such as incomplete stapling or excessive extrusion, and further leading to potential leakage and irregular stenosis of the anastomosis, which would greatly affect the surgical effect and is not helpful for postoperative recovery of the patient.

Therefore, there is an urgent requirement to improve the use stability and convenience of the stapler.

### SUMMARY OF THE INVENTION

An object of the present application is to overcome the deficiencies of the prior art and to provide a stapler with switch-lock linkage mechanism.

The object of the present application is achieved through the following technical solutions.

A stapler with switch-lock linkage mechanism includes a housing, a knob arranged at a front end of the housing, an effector driven by the knob and is at least axially rotatable, a drive motor arranged in the housing for driving the effector to complete anastomosis, a lead screw, and a firing locking push button inserted in the housing, wherein at least one end of the firing locking push button is protruded out of the housing. The firing locking push button can trigger a firing switch to provide an electrical signal to the drive motor. A front end of the firing locking push button has a driving part, and a trigger block that matches with a detection module of the firing switch is fixedly disposed on a bottom of the driving part. A push rod is arranged in the housing in parallel to the lead screw, the push rod has a first end and a second end, wherein the first end abuts against the driving part all the time. The firing locking push button is movable radially to drive the driving part to synchronously drive the push rod to move axially relative to the knob, such that the second end of the push rod is plugged into the knob to lock the knob; meanwhile, the trigger block leaves optical path of the detection module, thereby triggering the firing switch.

In some embodiments, a front end face of the driving part is centrally formed with an inwardly concave transition groove, wherein a bottom of the transition groove is a first step surface, and the front end face of the driving part is symmetrically distributed relative to the transition groove to form second step surfaces. The first step surface, the second step surfaces and an end face of the first end of the push rod are all planes. In an initial state, the first end of the push rod abuts on the first step surface, and the second end of the push rod is separated from the knob; and in a second state, the first end of the push rod abuts against the second step surface, and the second end of the push rod is plugged into the knob.

In some embodiments, a groove wall of the transition groove and the end face of the first end are inclined surfaces or curved surfaces matching with each other.

In some embodiments, the second end has an outwardly protruding engaging block, and an inner side wall of the knob is evenly distributed with a group of engaging grooves that can match with the engaging block.

In some embodiments, a fixing block is fixedly disposed on the push rod, and a spring is sleeved on the push rod, wherein two ends of the spring respectively abut against the fixing block and the housing, such that the first end of the push rod is pushed, via elastic force of the spring, to always vertically abut against the driving part.

In some embodiments, a rotatable reset key member is disposed above the firing locking push button, and a grooved member that is configured to cooperate with the reset key member is disposed on a top portion of the firing locking push button. The reset key member is rod-shaped, and its tail end, after rotated, can abut against an inner wall of the grooved member and thus drive the firing locking push button to its original position.

In some embodiments, the reset key member includes a connection seat for pivotal connection and a first rod portion and a second rod portion arranged at upper and lower sides of the connection seat, a width of the first rod portion is greater than that of the second rod portion, and a tail end of the second rod portion is configured to abut against the grooved member.

In some embodiments, front and rear ends of the grooved member are both formed with openings, and a diameter of a rear end opening gradually decreases toward a front end opening to form a V shape; and the tail end of the second rod portion can pass through the opening at the front end.

In some embodiments, a nut is disposed on the lead screw and is movable along the lead screw, and a protrusion is disposed on a side wall of the nut. The first rod portion is located on a moving trajectory of the protrusion, such that forward movement of the nut can cause the protrusion to drive the reset key member to rotate clockwise to be separated from the grooved member, and backward movement of the nut can cause the protrusion to drive the reset key member to rotate counterclockwise to abut against the inner wall of the grooved member.

In some embodiments, the detection module is one of a firing switch detector, a displacement sensor and a pressure detector.

Beneficial effects of the present disclosure mainly includes:
1. A linkage mechanism is formed between the firing locking push button, the push rod and the detection module, such that radial movement of the firing locking push button can simultaneously achieve triggering of the detection module and locking of the knob. Position of cutting assembly controlled by the knob may be limited by locking the knob, such that, when the driving mechanism is in a ready-to-fire state, the position of the cutting assembly is fixed, which ensures the use stability during the anastomosis process and avoids the knob from rotation to affect the anastomosis effect, and by replacing manual control with a mechanical structure, the use convenience of the stapler is improved.
2. A reset key member that can be driven by the nut to rotate is provided, which, after use of the stapler, may be driven through backward movement of the nut to reset the firing locking push button synchronously, without the need to manually control the reset of the firing locking push button, which is convenient and fast, improves the use fluency and continuity, saves the doctor's operation time, and improves the operating efficiency of the stapler.
3. Both ends of the firing locking push button protrude out of the housing, and the driving part and the grooved member are both in a symmetrical structure. In this way, the firing locking push button can be radially moved from either side to lock or unlock the knob, and switch the firing switch, which can meet operating habits of left-handed and right-handed people, and thus further improves ease of use.

### BRIEF DESCRIPTION OF THE DRAWINGS

Technical solutions according to the present disclosure are further described below in conjunction with the accompanying drawings, wherein:
FIG. 1 is a schematic diagram of an embodiment of the present disclosure;
FIG. 2 is a partial structural schematic diagram of an embodiment of the present disclosure in an initial state;
FIG. 3 is a partial schematic diagram of an embodiment of the present disclosure during resetting;
FIG. 4 is a partial structural schematic diagram of an embodiment of the present disclosure in a second state;
FIG. 5 is a partial schematic diagram of an embodiment of the present disclosure in a second state;
FIG. 6 is an enlarged schematic diagram of part A in FIG. 5;
FIG. 7 is a partial schematic diagram of an embodiment of the present disclosure during resetting;
FIG. 8 is an enlarged schematic diagram of part B in FIG. 7; and
FIG. 9 is a partial structural schematic diagram of an embodiment of the present disclosure.

### DETAILED DESCRIPTION OF THE EMBODIMENTS

The present disclosure will be described in detail below in conjunction with the embodiments illustrated in the accompanying drawings. However, the present disclosure is not limited to these embodiments, and any structural, methodological, or functional changes made by a person skilled in the art based on these embodiments are all within the protection scope of the present disclosure.

It should be noted that, orientations or positional relationships indicated by terms such as "center", "up", "down", "left", "right", "front", "back", "vertical", "horizontal", "inner", "outer" and the like used in descriptions of the technical solutions are based on orientations or positional relationships depicted in the drawings, and are only for the convenience of description and simplify the description, rather than indicating or implying that the device or element referred to must have specific orientation, or be constructed and operated in specific orientation, and therefore cannot be understood as limitations to the present disclosure. In addition, terms such as "first", "second" and "third" are used for descriptive purposes only, and shall not be understood as indicating or implying relative importance. Moreover, in the description of the technical solutions, with an operator as a reference, a direction close to the operator is proximal, and a direction away from the operator is distal.

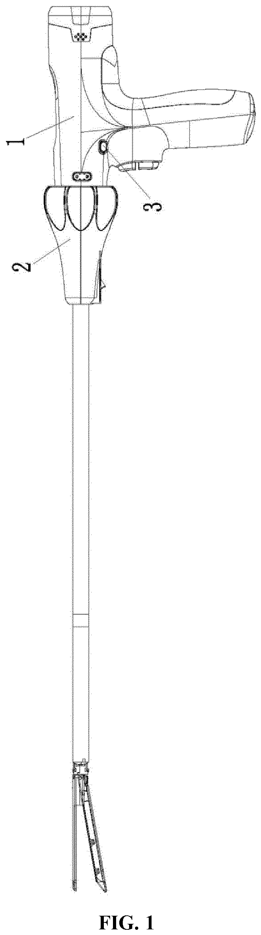

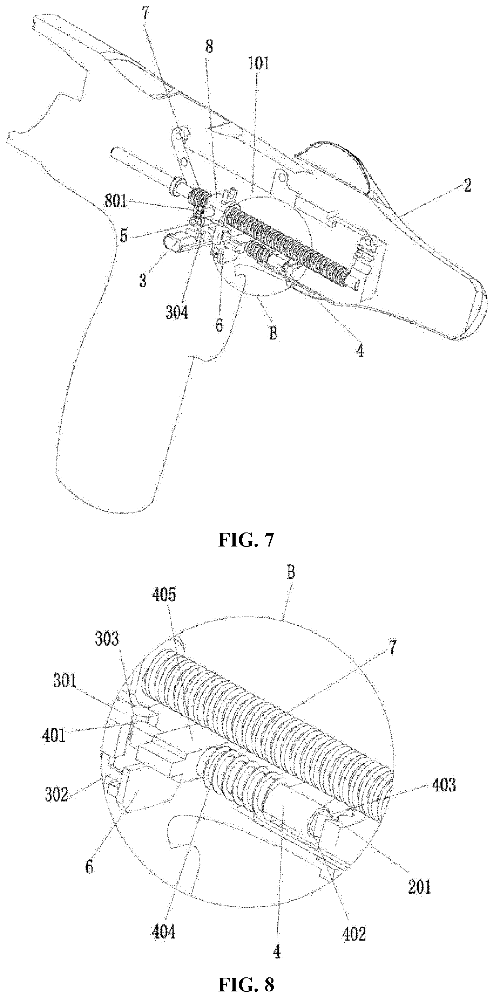

As shown in FIGS. 1 to 9, the present disclosure discloses a stapler with switch-lock linkage mechanism, including: a housing 1; a knob 2 disposed at a front end of the housing 1; an effector which is driven by the knob 2 and is at least capable of axial rotation; a drive motor disposed in the housing 1 for driving the effector to complete anastomosing operation; a lead screw 7; a firing locking push button 3, the firing locking push button 3 is inserted in the housing 1, at least one end of the firing locking push button is protruded from the housing 1, the firing locking push button 3 is configured to trigger a firing switch to provide an electrical signal to the drive motor, a front end of the firing locking push button 3 has a driving part 301, and a bottom of the driving part 30 1 is fixedly provided with a trigger block 302 matching with a detection module 6 of the firing switch; and a push rod 4 arranged parallel to the lead screw 7 in the housing 1, wherein the push rod 4 has a first end 401 and a second end 402, the first end 401 always abuts against the driving part 301, the firing locking push button 3 moves radially to drive the driving part 301 to synchronously drive the push rod 4 to move axially relative to the knob 2, such that the second end 402 of the push rod 4 is inserted into the knob 2 to lock the knob 2, and at the same time, the trigger block 302 leaves optical path of the detection module 6 to trigger the firing switch.

In some feasible embodiments, the detection module 6 may be one of a firing switch detector, a displacement sensor, and a pressure detector. In other feasible embodiments, the detection module 6 may be any detection module that can detect the displacement of the trigger block 302. The detection module 6 sends a firing signal or a closing signal to the firing switch by detecting the displacement of the trigger block 302.

In the present disclosure, a linkage structure is formed by the firing locking push button 3, the push rod 4 and the detection module 6 of the firing switch, such that triggering of the firing switch and locking of the knob 2 are simultaneously achieved through radial movement of the firing locking push button 3, and thus, when the driving mechanism is in a ready-to-fire state, the knob 2 remains in a locked state, thereby preventing the knob 2 from rotating during the firing state and affecting the stapling effect of the effector. Therefore, the surgical effect is ensured. On the other hand, there is no need to manually control the knob 2, which frees the doctor's hands and can relieve hand fatigue of the doctor, such that the doctor can hold it with both hands alternately to improve ease of use and stability.

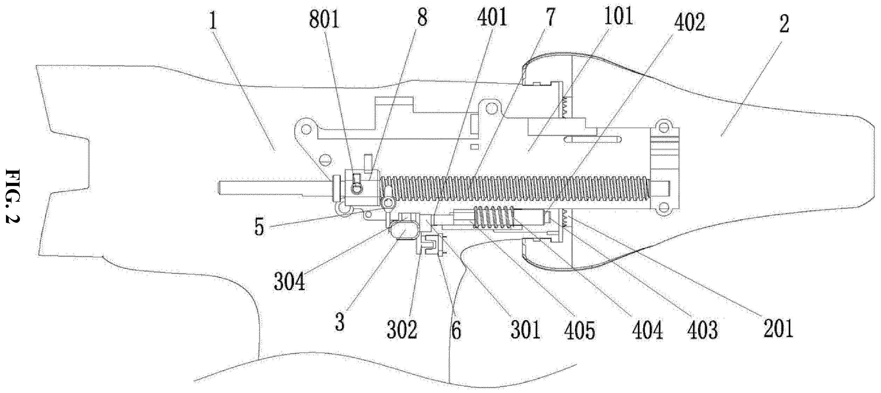

Specifically, as shown in FIGS. 2 to 4, the driving part 301 is a convex portion fixedly arranged at the front end of the firing locking push button 3, and an inwardly concave transition groove 303 is formed centrally on a front end face of the driving part 301, wherein a bottom of the transition groove 303 acts as a first step surface, and second step surfaces are formed by the front end face of the driving part 301, which are symmetrically arranged relative to the transition groove 303. The first step surface, the second step surfaces and end face of the first end 401 of the push rod 4 are all planar surfaces. In an initial state, the first end 401 of the push rod 4 abuts against the first step surface, and the second end 402 of the push rod is separated from the knob 2; in a second state, the first end 401 of the push rod 4 abuts against the second step surface, and the second end 402 is plugged into the knob 2.

In some embodiments, the driving part 301 may be integrally formed with the firing locking push button 3. In other embodiments, the driving part 301 may be other driving structure having spaced abutment surfaces, such as a structure of a continuous wave shape, a toothed shape, etc., which is not limited herein. The first step surface, the second step surfaces and the end face of the first end 401 of the push rod 4 are used as abutment surfaces, they are all planar surfaces, which may increase abutment area between the first end 401 and the first step surface or the second step surface to facilitate the abutment and reduce wear of the abutment surfaces during the abutment process. Of course, in other embodiments, abutment surfaces of the first end 401 and the first step surface and the second step surface may not be planar.

The arrangement of the transition groove 303 makes the front end face of the driving part 301 have a first step surface and a second step surface with a drop, to thereby drive the push rod 4 to move. Groove wall of the transition groove 303 is used for smooth transition between the first step surface and the second step surface, and the groove wall of the transition groove 303 and the end face of the first end 401 are inclined surfaces or arc surfaces matching with each other. In some embodiments, the groove wall of the transition groove 303 is an inclined surface extending obliquely and is symmetrically arranged to form a V-shape. The inclined arrangement of the side wall of the transition groove 303 can facilitate the first end 401 of the push rod 4 to move along the side wall of the transition groove 303 to the second step surface when the firing locking push button 3 moves radially, so as to improve smoothness of the axial movement of the push rod 4. Furthermore, inclined surfaces matching the side wall of the transition groove 303 are formed on both sides of the end face of the first end 401, such that the first end 401 can move more smoothly along the transition groove 303 and quickly transit between the first step surface and the second step surface. In other embodiments, the groove wall of the transition groove 303 may be a smooth arc surface, and the first end 401 may have an arc shape matching therewith, such that the first end 401 can move along the transition groove 303 to lock and reset the knob 2.

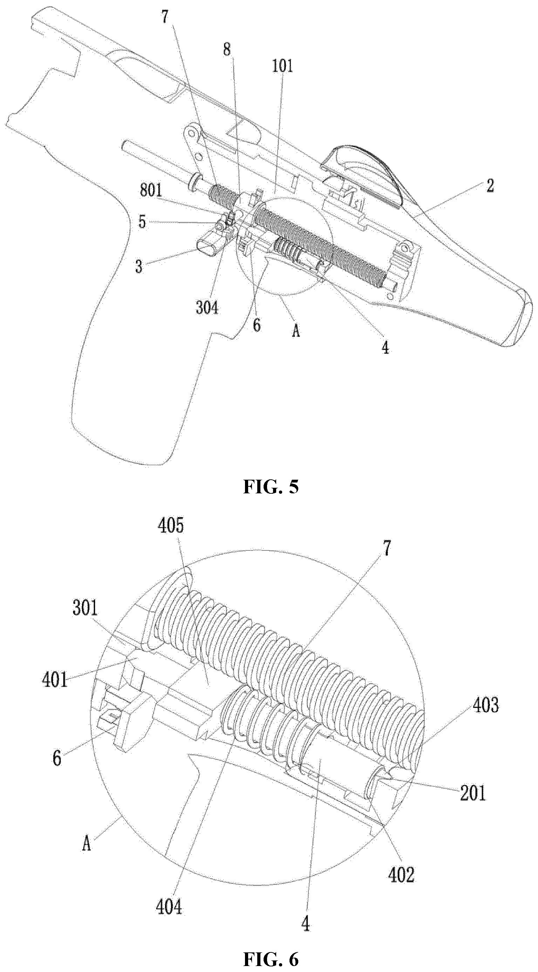

As shown in FIG. 5 to FIG. 8, the second end 402 has an outwardly protruding engaging block 403, and an inner side wall of the knob 2 is evenly distributed with a group of engaging grooves 201 that can engage with the engaging block 403. The engaging block 403 is engaged with the engaging groove 201, such that the push rod 4 can lock the knob 2, preventing the knob 2 from rotating to change an angle of the effector in the firing state, thereby ensuring stability and accuracy of the surgical operation.

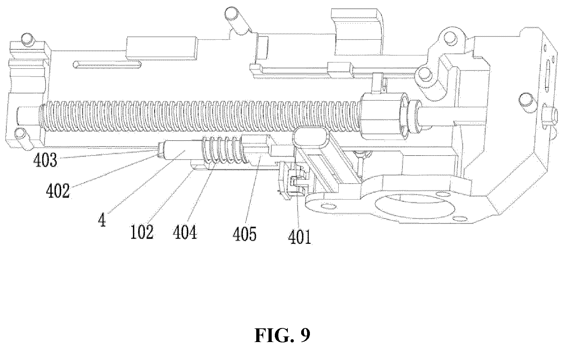

Further, a fixing block 405 is fixedly disposed on the push rod 4, and a spring 404 is sleeved on the push rod 4, and opposite ends of the spring 404 abut against the fixing block 405 and the housing 1, respectively, so as to push the first end 401 of the push rod 4 to always vertically abut on the driving part 301 through elastic force of the spring. The spring 404 has a pre-shrinkage to ensure that it applies sufficient driving force to the push rod 4 such that the first end 401 of the push rod 4 and the driving part 301 remain in contact. In other embodiments, the push rod 4 may also be driven to move axially by other force-applying mechanisms. As shown in FIG. 9, a limit block 102 is provided on the inner wall of the housing 1 to abut against one end of the spring 404, so as to achieve indirect connection with the housing 1. In other embodiments, other structures that can abut or fixedly connect with an end of the spring 404 may also be provided on the inner wall of the housing 1, such that the other end of the spring 404 directly abuts against the housing 1, which is not limited herein.

As shown in FIGS. 2, 3, 4, 5 and 7, a rotatable reset key member 5 is provided above the firing locking push button 3. A top potion of the firing locking push button 3 has a grooved member 304 that cooperates with the reset key member 5. The reset key member 5 is rod-shaped, and a tail end of the reset key member can abut against an inner wall of the grooved member 304 by means of rotation of the tail end of the reset key member and drive the firing locking push button 3 to return to its original position.

Specifically, the reset key member 5 includes a connection seat 501 for pivotally connection and a first rod portion 502 and a second rod portion 503 arranged at upper and lower sides of the connection seat 501. A width of the first rod portion 502 is greater than that of the second rod portion 503, and a tail end of the second rod portion 503 can abut against the grooved member 304. A connection plate 101 for connection is arranged in the housing 1, and the connection seat 501 is pivotally connected to the connection plate 101, such that the reset key member 5 can be rotated.

Front and rear ends of the grooved member 304 are both formed with openings, and a diameter of the rear end opening gradually decreases toward the front end opening, forming a V shape; the tail end of the second rod portion 503 can pass through the front end opening. With the V-shaped structure of the grooved member 304, the grooved member 304 has an inclined inner wall. After the second rod portion 503 abuts on the inner wall of the grooved member 304, the tail end of the second rod portion 503 can move along the inner wall of the grooved member 304 as the second rod portion 503 rotates, thereby pushing the grooved member 304 to move radially.

In some embodiments of the present disclosure, both sides of the housing 1 have through holes, and each through hole allows an end of the firing locking push button 3 to protrude outward, and the firing locking push button 3 can be pressed from either side to move radially. The V-shaped structure of the grooved member 304 makes the grooved member 304 symmetrical, such that when the firing locking push button 3 is moved radially from either end thereof, the grooved member 304 can be driven by the second rod portion 503 of the reset key member 5 to move radially in a reverse direction to reset the firing locking push button 3. Similarly, the driving part 301 has a symmetrical structure, such that the firing locking push button 3 can be moved radially from either side to switch between the first step surface and the second step surface so as to lock or unlock the knob, and to switch the firing switch. Such a configuration may be operated by both a left-handed operator and a right-handed operator, further improving the convenience of use.

The reset key member 5 according to this solution is driven to rotate by a driving mechanism, as shown in FIGS. 2, 3, 5 and 7. A nut 8 is disposed on the lead screw 7, the nut 8 is movable along the lead screw. A drive motor (not shown in the figure) drives the lead screw 7 to rotate. The nut 8 is threadedly connected to the lead screw 7 and is driven to move along the lead screw 7 through rotation of the lead screw 7. A side wall of the nut 8 is provided with a protrusion 801, and the first rod portion 502 is located on a moving track of the protrusion 801, such that when the nut 8 is moved forward, the protrusion 801 can drive the reset key member 5 to rotate clockwise to be separated from the grooved member 304; and when the nut 8 is moved backward, the protrusion 801 can drive the reset key member 5 to rotate counterclockwise to abut against the inner wall of the grooved member 304. Such a configuration allows that, after use, the nut 8 may be moved backward, thereby driving the reset key member 5 to synchronously drive the firing locking push button 3 to reset. There is no need to manually control the firing locking push button 3 to reset, and actions of ending the firing state, unlocking the knob 2, and placing the drive motor in a non-startable state may be performed automatically and synchronously, and thus the stapler can quickly return to its initial state, which is convenient and quick, improves the fluency and continuity of use, saves doctor's operating time, and improves operating efficiency of the stapler.

Since the width of the first rod portion 502 is greater than that of the second rod portion 503, the first rod portion 502 may have a greater strength. In some embodiments, the side of the first rod portion 502 in contact with the protrusion 801 may be a plane to increase its contact area with the protrusion 801, reduce the impact of the protrusion 801 on the first rod portion 502, and increase the service life of the reset key member 5.

In addition, the effector includes a jaw, a staple cartridge seat, an anvil, a steering tab, a blade, an actuating sleeve and other component that is used to perform cutting and stapling functions. These components are available from the prior art, which are not focus of this disclosure, and thus will not be elaborated herein.

The operation process of this solution includes:
First, holding the housing 1 to move the jaw at a front end of the effector to the part to be operated, and adjusting the jaw by rotating the knob 2 such that the jaw is aligned with the part to be operated. At this time, the firing locking push button 3 is in the initial state as shown in FIG. 2, the first end 401 of the push rod 4 abuts against the first step surface, and the second end 402 of the push rod 4 is away from the engaging groove 201 in the knob 2.

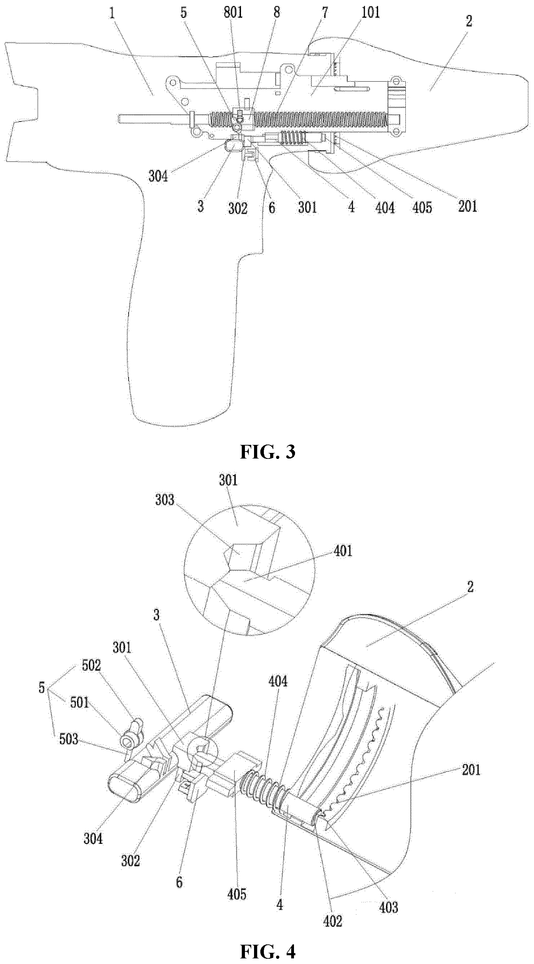

Then, pressing the firing locking push button 3 radially such that the firing locking push button 3 is in the second state as shown in FIG. 4. At this time, the first end 401 of the push rod 4 abuts against the second step surface, and the second end 402 of the push rod 4 is inserted into the engaging groove 201 in the knob 2 to lock the knob 2. At this time, the trigger block 302 is moved away from the optical path of the detection module 6, and the drive motor is in a startable state.

Next, pressing the firing switch to start the drive motor, which drives the lead screw 7 to rotate. The rotation of the lead screw 7 drives the nut 8 to move along it. A driving rod (not shown in the figures) is fixedly connected on the nut 8. A distal end of the driving rod is connected to the effector. The nut 8 drives the driving rod to move axially synchronously, such that axial movement of the driving rod drives the effector to perform the firing and stapling actions (which actions belong to the prior art and will not be described herein).

Finally, pressing the firing switch again, after the effector has completed the firing and stapling actions, to control the drive motor to rotate in a reverse direction, such that the lead screw 7 is rotated in a reverse direction, and then the nut 8 is moved toward a proximal end of the lead screw 7. FIGS. 3 and 5 show that the nut 8 is moving toward the proximal end of the lead screw 7, during which process, retreat of the nut 8 will drive the reset key member 5 to rotate counterclockwise, such that the tail end of the second rod portion 503 pushes the grooved member 304 to move radially, causing the firing locking push button 3 to return from the second state to the initial state, the first end 401 of the push rod 4 is moved from the second step surface to the first step surface, and the second end 402 of the push rod 4 is separated from the engaging groove 201 in the knob 2 to unlock the knob 2, and the trigger block 302 is again engaged in the detection module 6, blocking the optical path of the detection module 6, and thus the drive motor is in a non-startable state.

It should be understood that, although description is made herein according to the embodiments, it does not mean that each embodiment contains only one independent technical solution. Such kind of description is provided herein only for the sake of clarity. Those skilled in the art should consider the specification as a whole. Technical solutions according to various embodiments may also be appropriately combined to form other embodiment(s) that can be understood by those skilled in the art.

The series of detailed descriptions listed above are only descriptions of feasible embodiments of the present disclosure. They are not intended to limit the protection scope of the present disclosure. Any equivalent implementation methods or changes that do not deviate from the technical spirit of the present disclosure should fall within the protection scope of the present disclosure.

## Claims

1. A stapler with switch-lock linkage mechanism, comprising: a housing; a knob disposed at a front end of the housing; an effector driven by the knob and is at least axially rotatable; a drive motor disposed in the housing for driving the effector to complete anastomosis; a lead screw; and a firing locking push button inserted in the housing, wherein at least one end of the firing locking push button is protruded out of the housing, and the firing locking push button can trigger a firing switch to provide an electrical signal to the drive motor, **characterized in that**
a front end of the firing locking push button has a driving part, and a trigger block that matches with a detection module of the firing switch is fixedly disposed on a bottom of the driving part; a push rod is arranged in the housing in parallel to the lead screw, the push rod has a first end and a second end, wherein the first end abuts against the driving part all the time, the firing locking push button is movable radially to drive the driving part to synchronously drive the push rod to move axially relative to the knob, such that the second end of the push rod is plugged into the knob to lock the knob; and at the same time, the trigger block leaves and triggers the firing switch.

2. The stapler with switch-lock linkage mechanism according to claim 1. **characterized in that**, a front end face of the driving part is centrally formed with an inwardly concave transition groove, wherein a bottom of the transition groove is a first step surface, and the front end face of the driving part is symmetrically distributed with respect to the transition groove into second step surfaces; the first step surface, the second step surfaces and an end face of the first end of the push rod are all planes, wherein in an initial state, the first end of the push rod abuts against the first step surface, and the second end of the push rod is separated from the knob; and in a second state, the first end of the push rod abuts against the second step surface, and the second end of the push rod is plugged into the knob.

3. The stapler with switch-lock linkage mechanism according to claim 2, **characterized in that**, a groove wall of the transition groove and the end face of the first end are inclined surfaces or curved surfaces matching with each other.

4. The stapler with switch-lock linkage mechanism according to claim 2, **characterized in that**, the second end has an outwardly protruding engaging block, and an inner side wall of the knob is evenly distributed with a group of engaging grooves that match with the engaging block.

5. The stapler with switch-lock linkage mechanism according to claim 2, **characterized in that**, a fixing block is fixedly disposed on the push rod, and a spring is sleeved on the push rod, wherein two ends of the spring respectively abut against the fixing block and the housing such that the first end of the push rod is pushed, via elastic force of the spring, to always vertically abut against the driving part.

6. The stapler with switch-lock linkage mechanism according to any one of claims 1 to 5, **characterized in that**, a rotatable reset key member is disposed above the firing locking push button, and a grooved member that is configured to cooperate with the reset key member is disposed on a top portion of the firing locking push button; the reset key member is rod-shaped, and a tail end of the reset key member, after rotated, can abut against an inner wall of the grooved member and drive the firing locking push button to its original position.

7. The stapler with switch-lock linkage mechanism according to claim 6, **characterized in that**, the reset key member comprises a connection seat for pivotal connection, and a first rod portion and a second rod portion arranged at upper and lower sides of the connection seat, wherein a width of the first rod portion is greater than that of the second rod portion, and a tail end of the second rod portion is configured to abut against the grooved member.

8. The stapler with switch-lock linkage mechanism according to claim 7, **characterized in that**, front and rear ends of the grooved member are both formed with openings, and a diameter of a rear end opening gradually decreases toward a front end opening to form a V shape; and the tail end of the second rod portion can pass through the front end opening.

9. The stapler with switch-lock linkage mechanism according to claim 8, **characterized in that**, a nut is disposed on the lead screw and is movable along the lead screw, and a protrusion is disposed on a side wall of the nut; the first rod portion is located on a moving trajectory of the protrusion, such that forward movement of the nut can cause the protrusion to drive the reset key member to rotate clockwise to be separated from the grooved member, and backward movement of the nut can cause the protrusion to drive the reset key member to rotate counterclockwise to abut against the inner wall of the grooved member.

10. The stapler with switch-lock linkage mechanism according to claim 1, **characterized in that**, the detection module is one of a firing switch detector, a displacement sensor and a pressure detector.
